# EUROPÄISCHE PATENTSCHRIFT

(11) **EP 3 364 913 B1**
(45) Veröffentlichungstag und Bekanntmachung des Hinweises auf die Patenterteilung: **19.08.2020**
(21) Anmeldenummer: 17780020.8
(22) Anmeldetag: 15.09.2017
(51) Int. Cl.: A61C 13/00, A61C 13/08, A61C 13/083

(54) **VERFAHREN UND VORRICHTUNG ZUR FUNKTIONALISIERUNG DENTALER RESTAURATIONEN**
METHOD AND DEVICE FOR FUNCTIONALISING DENTAL RESTORATIONS
PROCÉDÉ ET DISPOSITIF DE FONCTIONNALISATION DE PROTHÈSES DENTAIRES

(30) Priorität: 15.09.2016 DE 102016117395
(43) Veröffentlichungstag der Anmeldung: 29.08.2018
(73) Patentinhaber: Bredent GmbH & Co. KG, 89250 Senden (DE)
(72) Erfinder: MEYER, Joachim, 89081 Ulm (DE)
(74) Vertreter: Baur & Weber Patentanwälte PartG mbB
(86) Internationale Anmeldenummer: PCT/EP2017/073257
(87) Internationale Veröffentlichungsnummer: WO 2018/050811

(56) Entgegenhaltungen:
- EP-A1- 1 153 002
- EP-A1- 1 911 568
- DE-A1-102011 117 005
- JP-A- 2005 059 477

## Beschreibung

Die Erfindung betrifft ein Verfahren zur Funktionalisierung dentaler Restaurationen , eine Appliziervorrichtung zur Funktionalisierung dentaler Restaurationen, ein Softwareprogrammprodukt und eine Steuereinrichtung.

Neuartige Herstellverfahren für permanente Restaurationen werden vor allem in Bezug auf keramische Werkstoffe und Keramik/Hybrid- Werkstoffe entwickelt. Desweiteren sind monolithische keramische Werkstoffe aus hochtransparentem Zirkoniumdioxid marktüblich. Diese Werkstoffe werden vollanatomisch in subtraktiven Verarbeitungsformen beispielsweise durch Spanen oder Schleifen sowie in additiven Verfahren beispielsweise mittels thermodynamischen Presstechniken formgebend hergestellt.

Aus der WO 2005/070322 A1 ist ein Verfahren zur Herstellung eines anorganisch-anorganischen Compositwerkstoffes bekannt, bei welchem aus einem Oxidkeramikpulver oder einem Pulver einer Oxidkeramikmischung nach formgebender Verarbeitung und Vorsintern ein offenporiges, kristallines Oxidkeramik-Formteil hergestellt wird, auf diese ein Infiltrationsstoff unter Vakuum und bei Raumtemperatur aufgebracht wird und bei Luftatmosphäre und Umgebungsdruck die Oxidkeramik zu einem anorganisch-anorganischen Compositwerkstoff verdichtend gesintert wird.

In der EP 1 911 568 A1 wird eine Vorrichtung zur schichtweisen Herstellung von Zahnersatz angegeben, bei der mittels einer Pulverauftrageinrichtung und einer Flüssigkeitsauftrageinrichtung auf einem Basisträger Schicht für Schicht ein Zahnersatz aufbaubar ist. Die Flüssigkeit kann dabei verschiedene Farbstoffe sowie Glanzstoffe enthalten. Der Auftragung kann ein digitales Modell eines Zahnersatzes zugrunde liegen.

Aus der DE 10 2011 117 005 A1 ist ein Verfahren zur Herstellung eines keramischen Formkörpers bekannt, bei dem ein Schlicker durch wiederholtes, lagenweises Abscheiden zum Aufbau eines keramischen Formkörpers verwendet wird. Einzelne Schichten können durch Abtragen von Teilbereichen geglättet werden, so dass ortsselektiv mit einer Tintenflüssigkeit Farbpigmente in die Schlickerschicht eingebracht werden können. Die Schichtauftragung sowie die Einfärbung einzelner Flächenbereiche folgen einem dreidimensionalen Modell.

In der JP 2005 059 477 A, wird ein Abgabemechanismus von Flüssigkeiten beschrieben, der eine vorgegebene Menge einer Flüssigkeit auf Puder abgibt, worauf-hin eine Halogenlampe eine Polymerisation einleitet. Die Daten über Beleuchtung, Puderabgabe und Flüssigkeitsabgabe stammen aus einem Controller, der einen Datensatz über die dreidimensionale Struktur des Objekts umfasst.

Bekannt ist es außerdem, dass Weißlinge aus Zirkoniumdioxid vordem Dichtsintern mit einer metalloxidhaltigen Flüssigkeit infiltriert werden, um so eine individuelle Farbgebung zu erreichen. Ein anderer Ansatz ist, dass Halbzeuge aus polychromen Gradienten bereitgestellt werden, um den Farbverlauf eines natürlichen Zahn nachzuahmen.

So ist in der EP 1 153 002 A1 das Einfärben von Keramiken mittels ionischer oder komplexhaltiger Lösungen beschrieben. Hierfür geeignete Lösungen enthalten definierte Konzentrationen mindestens eines der Salze oder Komplexe der Seltenerden-Elemente oder der Elemente der Nebengruppen.

In der WO 2014/206439 A1 ist ein Verfahren zum Färben von gebinderter und/oder entbinderter und/oder angesinterter und/oder durchgängig poröser Keramik beschrieben, insbesondere von porösen Keramikformkörpern, die insbesondere in der Dentaltechnik Anwendung finden, wobei der farbgebende Auftrag mit einem Auftragungs- und/oder stiftartigen Werkzeug, das Verbundmaterial, insbesondere Malstiftverbundmaterial, versetzt mit farbgebenden Komponenten und/oder brandfesten Pigmenten und/oder Oxiden und/oder färbenden und fluoreszierenden Metalloxiden und/oder organischen oder anorganischen Salzen, aufweist, erfolgt.

Nachteilig in der bekannten Infiltrationstechnik ist, dass diese Flüssigkeiten von Hand aufgetragen werden und durch die Materialabsorption nicht genau definiert werden kann, wo die Farbgradienten liegen oder Trennungen sind und wie stark die Infiltration die Farbsättigung steuert, da diese Flüssigkeiten die gewünschte Farbentwicklung bezüglich Intensität und Farbtiefe (Chroma) erst durch die thermische Behandlung des Dichtsinterns erfährt. Aufgrund der sehr unterschiedlichen Farbbereiche einer Krone/Brücke ist eine differenzierende Infiltration mit genauer Farbgestaltung nicht möglich. Die anatomisch bedingt sehr unterschiedlichen Materialstärken innerhalb einer Krone/Brücke führen zu stark unterschiedlichen Absorptions- und Sättigungsverhalten. Aufgrund dieser Eigenschaft können geeignete Infiltrationen oder Funktionalisierungen von Oberflächen oder Massenbereichen nicht genau definiert werden.

Die oben erwähnte Produktlösung der polychromen Schichtung von Halbzeugen hat den Nachteil, dass bei einem konkaven Okklusionsverlauf von Kronen Brücken die Farbgradienten im Halbzeug nicht gekrümmt sondern horizontal verlaufen. Dadurch ergeben sich ungleichmäßige, unnatürlich Farbverläufe.

Die gemeinsame Problematik dieser unterschiedlichen Produktlösungen ist, dass eine reproduzierbare, individuelle Farbgestaltung nicht möglich ist. Ein weiterer Nachteil bei den eingefärbten Halbzeugen ist, dass der Kunde einen Farbschlüssel von typischerweise mindestens sechzehn polychromatischen Zahnfarben reproduzieren und daher Halbzeuge entsprechend den jeweiligen Anforderungen einsetzen muss, was sehr zeitintensiv ist und eine sehr hohe Lagerhaltung verlangt.

In der DE 10 2010 037 160 A1 wird ein Verfahren zur Herstellung von Zahnersatz beschrieben, bei dem zur Herstellung einer Verblendung auf einem Trägergerüst in Abhängigkeit eines digitalen Modells des Zahnersatzes mehrere Schichten eines Materialgemisches aufgetragen werden. Dabei können bestimmte Schichten der unmittelbar aufeinanderfolgend aufgetragenen Schichten auch eine Einfärbung aufweisen. Zur Ausrichtung bzw. Positionierung des Trägers ist eine Aufnahme vorgesehen, die in Abhängigkeit von Steuerbefehlen positioniert wird.

In der US 2010/0260924 A1 wird ein Digitaldruckprozess beschrieben, der zur Funktionalisierung einer Oberfläche von Zahnersatz herangezogen wird. Hierbei kann eine mehrfarbige Oberfläche hergestellt werden, die den ursprünglichen Zähnen farblich angepasst wird.

Bei diesen Verfahren scheint jedoch es nachteilig zu sein, dass es in der Fertigung zu komplexen Schicht- bzw. Massenaufträgen kommt, die das Volumen verändern und komplexe Produktionssysteme sowie Produktionszeiten benötigen. Desweiteren kommt es durch den oberflächlichen Materialauftrag (z.B. der Farbe) zu einer Oberflächenvergrößerung, die später von Hand den Okklusionsverhältnissen angepasst werden muss. Hier sind durch die notwendigen Korrekturen wiederum ein Abtrag der oberflächlichen Farbschicht zu erwarten.

Aufgrund der fortschreitenden Rapid-Prototyping-Verfahren sind andere Materialeigenschaften und Farbgebungssysteme gefordert, um hier mit einer sehr kleinen Auswahl an Grundmaterial/Farben ein Maximum an Produktion und farbsicherer und individueller Reproduktion zu erzielen.

Es ist daher Aufgabe der Erfindung, ein Verfahren zur Funktionalisierung dentaler Restaurationen und eine Vorrichtung zur Funktionalisierung dentaler Restaurationen zu schaffen, die eine vielfältige Modifikation von Oberflächen erlauben.

Diese Aufgabe wird durch die unabhängigen Patentansprüche 1 und 10 gelöst. Weitere vorteilhafte Ausgestaltungen der Erfindung sind jeweils Gegenstand der Unteransprüche. Diese können in technologisch sinnvoller Weise miteinander kombiniert werden. Die Beschreibung, insbesondere im Zusammenhang mit der Zeichnung, charakterisiert und spezifiziert die Erfindung zusätzlich.

Gemäß der Erfindung wird in einem ersten Aspekt ein Verfahren zur Oberflächenfunktionalisierung dentaler Restaurationen angegeben, bei dem folgende Schritte ausgeführt werden: Einsetzen eines Formkörpers in eine Aufnahme einer Appliziervorrichtung, Bereitstellen eines digitalen Datensatzes, der wenigstens einen Teilbereich der Oberfläche des dentalen Formkörpers repräsentiert, Auswählen einer Funktionalisierungsfläche innerhalb des Teilbereichs der Oberfläche des dentalen Formkörpers, Infiltration eines Volumens unter der Funktionalisierungsfläche mit einem Funktionalisierungswirkstoff, wobei die Infiltration mit dem Funktionalisierungswirkstoff durch gezieltes Bewegen der Aufnahme und/oder einer Abgabevorrichtung erfolgt und die Infiltration eine Massezunahme des Formkörpers bei im wesentlichen unveränderten Volumen des Formkörpers bewirkt, und Entnahme des dentalen Formkörpers aus der Appliziervorrichtung, und Aktivieren des Funktionalisierungswirkstoffs in einem thermischen, optischen oder chemischen Vorgang.

Demnach ist es erfindungsgemäß möglich, dreidimensionale Oberflächen digital zu erfassen und zu bearbeiten, wodurch einer Funktionalisierung im Beschichtungsverfahren mittels geeigneter Wirkstoffe ermöglicht wird, um die Oberfläche entsprechend aufzubereiten. Dabei können bereits markübliche Produktionsverfahren und Werkstoffe nachträglich digital weiterverarbeitet werden. Die erfindungsgemäße Vorgehensweise erlaubt es, die Oberfläche digital zu bearbeiten und die hierbei verwendeten Funktionalisierungsstoffe in den Volumenkörper zwecks Änderung der optischen, mechanischen oder physikalischen Eigenschaften zu absorbieren.

Das hat den Vorteil, dass innerhalb der Rapid-Prototyping-Verfahren keine kleinen Produktionsbatches zusammengestellt werden müssen, was hinsichtlich der Leistungsfähigkeit und Produktionskapazität und technischer Auslegung dieser Systeme nicht wirtschaftlich wäre. Für den Formkörper kann keramisches Basismaterial gedruckt, gepresst oder gefräst werden, unabhängig davon, welche spätere individuelle Funktionalisierung der jeweiligen Zahnrestauration in Form einer Krone oder Brücke gewünscht ist. Der Vorteil hier ist, dass im Rapid-Prototyping-Verfahren nun große Produktionsbatches vorproduziert werden können, die anschließend einer individuellen Funktionalisierung unterzogen werden.

Im Gegensatz zu einem reinen oberflächlichen Farbauftrag, wie er bereits im Stand der Technik beschrieben wurde, wird hier eine digital geführte Oberflächenfunktionalisierung mittels Infiltration von Keramiken durchgeführt, wobei hier kein oberflächlicher Auftrag unter Volumenzunahme von Schichten sondern ein Absorbieren des Funktionalisierungswirkstoffs unter Massezunahme erfolgt, wodurch diese Absorption die infiltrierte Schicht/Masse mit den gewünschten Eigenschaften funktionalisiert.

Gemäß einer Ausführungsform der Erfindung wird der Formkörper als poröse Keramik, offene Keramik, Weißkeramik, Grünkeramik, Grünling oder monolithischer Keramik bereitgestellt oder aus daraus bestehendem Halbzeug verarbeitet, mittels eines Pressverfahrens, mittels eines formgebenden Gießverfahrens oder in einem formfreien 3D-Druckverfahren hergestellt.

Erfindungsgemäß wird eine automatisierte, maschinelle Verarbeitung von bereits verarbeiteten Keramiken, d.h. von zahntechnische Restaurationen, die bereits aus einem Halbzeug weiterverarbeitet wurden oder in einem Pressverfahren hergestellt wurden, vorgenommen.

Dabei kann gemäß einer weiteren Ausführungsform der Erfindung als Funktionalisierungswirkstoff ein Farbauftrag, ein lichthärtender Lack, ein Kaltsilan, eine Harzinfiltration oder ein oberflächlicher Auftrag von keramischen oder polymeren Lasuren, Metalloxiden oder seltene Erden zur Veränderung der optischen Eigenschaften applizierbar sein.

Über die Oberfläche kann ein geeigneter Funktionalisierungswirkstoff mittels Infiltration in das darunterliegende Volumen eingebracht werden, wobei beispielsweise ein oberflächlicher Auftrag auf bereits dichtgesinterte und gebrannte Strukturen oder auf teil- oder vollpolymerisierte Polymere denkbar ist. Dazu gehört das Applizieren von optischen Veränderungen wie Helligkeit oder Opaleszenz, chemische Funktionalitäten hinsichtlich Optimierung von Haftverbunden, physikalische Eigenschaften hinsichtlich Optimierung von Werkstoffeigenschaften mittels Oberflächenbeschichtung und Infiltration wie Härte, Elastizität oder die Veränderung des Wärmeausdehnungskoeffizienten durch Eintrag von Leuzit in Keramiken.

Zur Farbanpassung ist es beispielsweise möglich, im Rapid-Prototyping-Verfahren nun große Produktionsbatches vorzuproduzieren, ohne diese wie bisher in kleine Produktionsbatches anhand von Aufteilung der sechzehn polychromen Zahnfarben zu sortieren. Durch die Erfindung ist es jetzt möglich, einen gemeinsamen Produktionsbatch unabhängig der anschließenden unterschiedlichen und individuellen Funktionalisierung auch von Teilbereichen zusammenzulegen und zu produzieren, die anschließend einer individuellen Funktionalisierung im Beschichtungsverfahren unterzogen werden.

Mittels eines CMYK basierten Farbsystems wäre es möglich, alle Zahnfarben herzustellen. Die aktuellen dentaltypischen Farbsysteme setzen sich hier aus dem klassisch angelehnten oder abgeleiteten Zahnfarbzuordnungen "VITA classical"-Farbsystem zusammen. Die Gruppierung der Farben im "VITA classical"-Farbsystem erfolgt nach Farbtönen und deren Verteilung im Zahnfarbraum: A1-A4 Rötlich-bräunlich, B1-B4 Rötlich-gelblich, C1-C4 Grautöne und D2-D4 Rötlich-grau. Ein CMYK basiertes Farbsystem wäre daher an die Drucktechnik angelehnt, wobei Farben geräteunabhängig beschrieben werden können.

Gemäß einer weiteren Ausführungsform der Erfindung wird die Abgabevorrichtung der Appliziervorrichtung als Druckkopf, als Sprühkopf oder als eine auf kontaktbehaftetem Drucken basierende Applikationseinheit ausgeführt.

Dies ermöglicht das Auftragen des flüssigen Funktionalisierungswirkstoffes ähnlich zu einem Drucker. Dabei kann die Flüssigkeit sowohl aufgedruckt als auch aufgesprüht oder kontaktbehaftet bedruckt werden. Durch Steuerung der Abgabevorrichtung an der Appliziervorrichtung kann lokal die gewünschte Menge oder Stärke aufgebracht werden. Das Auftragen des flüssigen Funktionalisierungswirkstoffes führt zu einem Eindringen in den Formkörper, so dass dieser zwar eine Masse- jedoch keine Volumenzunahme erfährt.

Gemäß einer weiteren Ausführungsform der Erfindung wird der digitale Datensatz mittels einer Raumüberführung und lagerichtiger Zuordnung in die Appliziervorrichtung übernommen.

Vorteilhaft ist hier, dass der zum physikalisch Formkörper erstellte digitale Oberflächendatensatz, der beispielsweise vorab extern erfasst wurde, mittels einer Raumüberführung und lagerichtiger Zuordnung der unterschiedlichen Raumkoordinatensysteme der externen Messvorrichtung in die Appliziervorrichtung übernommen wird. So könnte zum Beispiel eine Triangulation eines physikalischen Körpers vorgenommen werden, aus der dann die entsprechenden Steuerbefehle für die Appliziervorrichtung abgeleitet werden.

Gemäß einer weiteren Ausführungsform der Erfindung wird der digitale Datensatz über eine interne oder externe Messvorrichtung bestimmt, die taktil, optisch oder akustisch die Oberfläche des dentalen Formkörpers erfasst.

Neben einer Messvorrichtung, die auf unterschiedliche physikalische Weise eine Oberflächenbestimmung durchführen kann, ist es erfindungsgemäß auch vorgesehen, die Messvorrichtung als externe Einheit oder aber innerhalb der Appliziervorrichtung vorzusehen.

Gemäß einer weiteren Ausführungsform der Erfindung ist die Messvorrichtung als Kamera oder als dreidimensionaler Scanner ausgebildet.

Mittels einer Kamera oder eines Scanners kann die Oberfläche des Formkörpers erfasst werden. Eventuell könnte auch keine Kamera zur Oberflächenerfassung vorhanden sein, aber mittels Nullspanvorrichtung diese Flächendatensätze einer separaten Vermessungseinheit zur Oberflächenerfassung in Nullpunktzuordnung des Scanners als Daten an die Appliziervorrichtung übergeben werden.

Gemäß einer weiteren Ausführungsform der Erfindung wird zusätzlich auf der Oberfläche des Formkörpers eine Farbmessung mittels eines Digitalsystems durchgeführt.

Eine optische Messvorrichtung zur Oberflächenerfassung wird typischerweise eine Optik aufweisen, die für die Farbmessung nicht vorgesehen oder dazu optimiert ist. Demnach müsste eine spezielle Optik installiert sein, die von der zur dreidimensionalen Erfassung der Oberfläche verwendeten Optik getrennt ist.

Gemäß einer weiteren Ausführungsform der Erfindung erfolgt eine Entfernungsmessung, um einen Abstand während des Auftragens des Funktionalisierungswirkstoffes zu einer Abgabevorrichtung zu justieren oder einen Referenzpunkt zu bestimmen.

Die Bestimmung des Referenzpunktes ermöglicht darüber hinaus auch eine Positionierung der dentalen Restauration oder des Trägers zum Nullpunkt.

Gemäß einer weiteren Ausführungsform der Erfindung kann die Appliziervorrichtung ausgehend von dem digitalen Datensatz definierte Flächen ansteuern und das Auftragen des Funktionalisierungswirkstoffes bezüglich der verwendeten Materialien, deren Stärken oder Dichten steuern.

Dabei kann das Auftragen innerhalb einer fünfachsigen Bearbeitung in allen drei Raumachsen koordiniert werden, um hier auch Hinterschnitte auf der dentalen Restauration zu erreichen.

In einem zweiten Aspekt wird eine Appliziervorrichtung zur Durchführung eines Verfahrens zur Funktionalisierung dentaler Restaurationen angegeben, insbesondere nach Verfahren wie oben beschrieben, umfassend eine Auflage zur Aufnahme eines teilbearbeiteten dentalen Formkörpers für die Herstellung einer dentalen Restauration und eine Abgabevorrichtung zum wenigstens abschnittsweisen räumlich vordefinierten Infiltrieren eines flüssigen Funktionalisierungswirkstoffs in einem Volumen unter einer über einen digitalen Datensatz zugeordnete Oberfläche des dentalen Formkörpers, wobei die Infiltration eine Massezunahme des Formkörpers bei im wesentlichen unveränderten Volumen des Formkörpers bewirkt.

Die Appliziervorrichtung ermöglicht das Auftragen eines Funktionalisierungswirkstoffes in einem vorgegebenen Flächenbereich, wobei das infiltrierte Volumen, die Schichtstärke und andere Parameter beim Auftrag präzise steuerbar sind. Dazu wird ein digitaler Datensatz bereitgestellt, der als Grundlage zum Auftragen eines flüssigen Funktionalisierungswirkstoffs auf eine über einen digitalen Datensatz zugeordnete Oberfläche des dentalen Formkörpers dient.

Gemäß einer weiteren Ausführungsform der Erfindung ist die Auflage entlang mehrerer Achsen bewegbar, vorzugsweise entlang einer ersten Achse und einer zweiten Achse verschiebbar und entlang der zweiten Achse und/oder einer dritten Achse drehbar, und erfolgt das Auftragen des Funktionalisierungswirkstoffs durch gezieltes Bewegen der Auflage. Dabei kann die Abgabevorrichtung entlang wenigstens einer weiteren Achse bewegbar sein und das Auftragen des Funktionalisierungswirkstoffs durch gezieltes Bewegen der Abgabevorrichtung erfolgen.

Demnach wird eine Möglichkeit geschaffen, eine individuell definierbare Flächenauswahl auf der Oberfläche des dentalen Formkörpers zu treffen und diese maschinell mit reproduzierbaren Parametern zu bearbeiten, in dem ein definierter Mengenauftrag auf definierten Flächen durch das Auftragen des Funktionalisierungswirkstoffs erfolgt. So sind beispielsweise die zu erzielenden Homogentäten hinsichtlich auftragender Schichtstärke oder die Materialdichte und Materialstärke des Funktionalisierungswirkstoffs präzise steuerbar, so dass aufgrund der Materialeigenschaften des Funktionalisierungswirkstoffs keine unterschiedlichen Ergebnisse nach Infiltration in das darunterleigende Volumen hervorgebracht werden, wie dies bei einer nicht gesteuerten analogen Anwendung durch manuelles Auftragen zu erwarten wäre. Mittels einer digitalen Verarbeitung können individuell Materialeigenschaften als Bearbeitungsparameter (Werkstoff, Dichte, Materialstärke) des Funktionalisierungswirkstoffs größtenteils berücksichtigt und damit kompensiert werden.

Des Weiteren wird in Anspruch 14 ein Softwareprogrammprodukt angegeben, das für einen Prozessor lesbare Befehle enthält, die geeignet sind, ein oben beschriebenes Verfahren auszuführen.

Schließlich wird in Anspruch 15 eine Steuereinrichtung einer Appliziervorrichtung angegeben, die aus einem Speicher Befehle erhält, die geeignet sind, ein oben beschriebenes Verfahren auszuführen.

Nachfolgend werden einige Ausführungsbeispiele anhand der Zeichnung näher erläutert. Es zeigen:
Fig. 1 eine perspektivische Seitenansicht einer Appliziervorrichtung zur Anwendung des erfindungsgemäßen Verfahrens, und
Fig.2 eine Detailansicht der Appliziervorrichtung aus Fig. 1.

In den Figuren sind gleiche oder funktional gleich wirkende Bauteile mit den gleichen Bezugszeichen versehen.

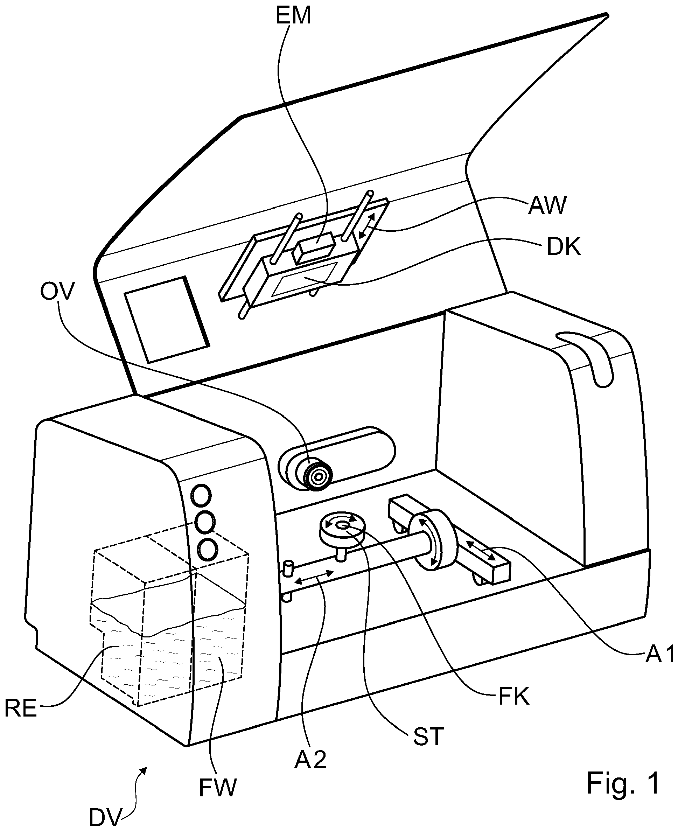

In Fig. 1 ist in einer perspektivischen Seitenansicht eine dreidimensionale Repräsentation einer Appliziervorrichtung DV zur Oberflächenfunktionalisierung für dentale Anwendungen gezeigt. Die Appliziervorrichtung DV weist eine Auflage in Form eines Scantisches ST auf, die entlang einer Achse senkrecht zur Auflagefläche drehbar ist. Desweiteren ist der Scantisch ST entlang einer als X-Achse vorgesehenen ersten Achse A1 und einer als Y-Achse vorgesehenen zweiten Achse A2 verschiebbar. Desweiteren kann der Scantisch um die zweite Achse A2 ebenfalls drehbar sein. Das Verschieben entlang der ersten Achse A1 und der zweiten Achse A2 sowie das Drehen um die zweite Achse A2 bzw. senkrecht zur Aufnahmefläche kann mittels geeigneter Stellmotoren (nicht in Fig. 1 gezeigt) gesteuert werden.

Auf den Scantisch ST wird ein Formkörper FK aufgebracht. Der Formkörper FK kann eine anatomische Außenfläche aufweisen oder als Gerüst bereitgestellt werden. Generell gilt, dass der Formkörper FK zur Herstellung einer dentalen Restauration verwendet wird. Dabei ist der Formkörper FK typischerweise aus einem Halbzeug verarbeitet oder mittels eines Pressverfahrens hergestellt.

Der Formkörper FK wird mittels einer Messvorrichtung OV dreidimensional erfasst, so dass die Oberfläche des Formkörpers FK digital weiterverarbeitet werden kann. Dabei kann das Erfassen der Oberfläche optisch aber auch taktil oder akustisch erfolgen. Bei akustischer Vermessung mittels Sonar oder Schallwellen werden weder Bewegungen noch mehrere Raumachsen benötigt. Das Erfassen der Oberfläche des Formkörpers kann aber auch außerhalb der Messvorrichtung OV mit einem dafür geeigneten Gerät erfolgen.

Zum abschnittsweisen Auftragen eines Funktionalisierungswirkstoffes, der beispielsweise einem Reservoir RE entnommen werden kann, ist eine Abgabevorrichtung in Form eines Druckkopfes DK vorgesehen, die mit dem Reservoir RE zur Zuführung des Funktionalisierungswirkstoffes FW in Verbindung steht. Der Druckkopf DK kann ebenfalls entlang einer weiteren Achse AW, die beispielsweise senkrecht zur ersten Achse A1 und senkrecht zur zweiten Achse A2 als Z-Achse gewählt sein kann. Die Achsen können auch ganz anders angeordnet sein. So muss die Kamera nicht am Deckel befestigt sein und kann anders angebracht sein, dasselbe gilt für den Druckkopf. Dabei kann das Auftragen innerhalb einer fünfachsigen Bearbeitung in allen drei Raumachsen koordiniert werden, um hier auch Hinterschnitte auf der dentalen Restauration zu erreichen.

Das Auftragen des Funktionalisierungswirkstoffes FW erfolgt nun durch gezieltes Bewegen des Scantisches ST sowie gegebenenfalls des Druckkopfes DK. Die Bewegungen sowohl des Druckkopfes DK als auch des Scantisches ST können dabei mittels einer geeigneten Steuervorrichtung koordiniert werden, so dass ein gewünschter Teilabschnitt auf der Oberfläche des Formkörpers FK mit dem Funktionalisierungswirkstoff FW versehen wird. Der Druckkopf DK kann typischerweise als Tintenstrahlkopf oder als Sprühkopf ausgeführt sein.

Generell kann die Oberfläche kann mit einem geeigneten Funktionalisierungswirkstoff FW beschichtet werden, wobei beispielsweise ein oberflächlicher Auftrag auf bereits dichtgesinterte und gebrannte Strukturen oder auf teil- oder vollpolymerisierte Polymere denkbar ist. Dazu gehört das Applizieren von optischen Veränderungen wie Helligkeit oder Opaleszenz, chemische Funktionalitäten hinsichtlich Optimierung von Haftverbunden, physikalische Eigenschaften hinsichtlich Optimierung von Werkstoffeigenschaften mittels Oberflächenbeschichtung und Infiltration wie Härte, Elastizität oder die Veränderung des Wärmeausdehnungskoeffizienten durch Eintrag von Leuzit in Keramiken. Das Auftragen des flüssigen Funktionalisierungswirkstoffes führt zu einem Eindringen in den Formkörper, so dass dieser zwar eine Masse- jedoch keine Volumenzunahme erfährt.

Die Messvorrichtung kann als Kamera oder als dreidimensionaler Scanner, beispielsweise als Laserscanner oder Streifenlichtscanner ausgebildet sein. Die Messvorrichtung kann auch eine taktile oder akustische Einheit anstelle der zusätzlich zur Kamera aufweisen.

Desweiteren ist ein Entfernungsmesser EM vorgesehen, der einen Abstand zwischen dem Druckkopf DK und dem Formkörper FK bestimmt. Der Entfernungsmesser EM kann dabei während des Auftragens des Funktionalisierungswirkstoffes FW den Abstand zum Druckkopf DK kontrollieren bzw. justieren aber auch zur Bestimmung eines Referenzpunktes, beispielsweise eines Nullpunktes oder Startpunktes zu Beginn des Auftragens oder zu Beginn des Abtastens der Oberfläche des Formkörpers FK genutzt werden.

Desweiteren ist es vorgesehen, die Messvorrichtung OV mit einer Farbmessung zu erweitern. Für die Farbmessung müsste eine spezielle Optik installiert sein, die von der zur dreidimensionalen Erfassung der Oberfläche verwendeten Optik getrennt ist.

Die Messvorrichtung OV ist vorzugsweise mit einer entsprechenden Bearbeitungssoftware gekoppelt, die die Oberfläche des Formkörpers FK nach der dreidimensionalen optischen Erfassung entsprechend darstellt. Die Oberfläche des Formkörpers FK kann nun innerhalb der Bearbeitungssoftware weiter verarbeitet werden, wobei die Weiterverarbeitung von der gewünschten Funktionalisierung abhängt. Beispielsweise ist es möglich, die Oberfläche des Formkörpers FK individuell digital zu kolorieren, wobei hier das weiter oben erwähnte Digitalsystem zur Farbmessung Anwendung findet.

Nach der digitalen Bearbeitung der Oberfläche wird der Druckvorgang mittels des Druckkopfes DK gestartet. Dabei ist es auch denkbar, sukzessive unterschiedliche Funktionalisierungswirkstoffe anzuwenden.

Nachdem der Druckprozess abgeschlossen ist, wird der bedruckte Formkörper FK der Appliziervorrichtung DV entnommen und in einem anschließenden thermischen, optischen oder chemischen Vorgang aktiviert.

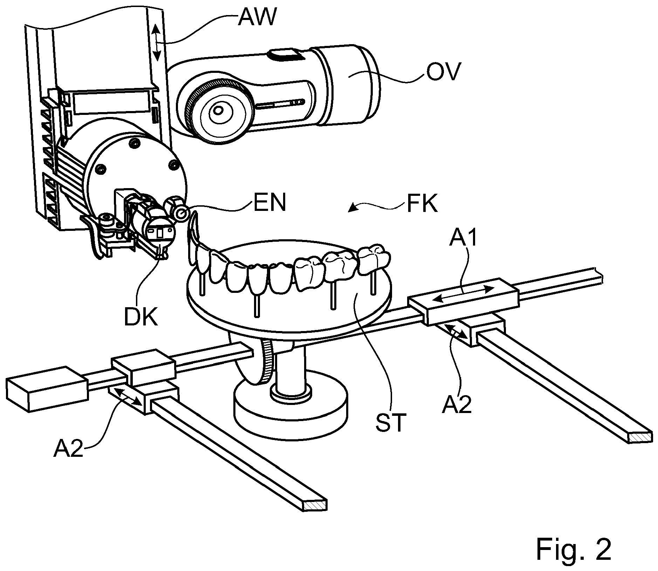

Ein Detail der Appliziervorrichtung DV zur Oberflächenfunktionalisierung ist in Fig. 2 gezeigt. Man erkennt, dass die Appliziervorrichtung DV die Auflage in Form eines Scantisches ST auf, die entlang einer Achse senkrecht zur Auflagefläche drehbar ist. Auf dem Scantisch ist eine dentale Restauration FK abgelegt.

Der Scantisch ST ist entlang der als X-Achse vorgesehenen ersten Achse A1 und der als Y-Achse vorgesehenen zweiten Achse A2 verschiebbar. Desweiteren kann der Scantisch um die zweite Achse A2 ebenfalls drehbar sein. Das Verschieben entlang der ersten Achse A1 und der zweiten Achse A2 sowie das Drehen um die zweite Achse A2 bzw. senkrecht zur Aufnahmefläche kann mittels geeigneter Stellmotoren (nicht in Fig. 2 gezeigt) gesteuert werden. Generell gilt, dass der Formkörper FK zur Herstellung einer dentalen Restauration verwendet wird. Dabei ist der Formkörper FK typischerweise aus einem Halbzeug verarbeitet oder mittels eines Pressverfahrens hergestellt.

Der Formkörper FK wird mittels der Messvorrichtung OV erfasst, um seine dreidimensionale Oberfläche zu bestimmen. Zusätzlich kann auf der Oberfläche des Formkörpers FK eine Farbmessung mittels eines Digitalsystems (nicht in Fig. 2 gezeigt) durchgeführt werden. Des Weiteren erfolgt über einen Entfernungs- oder Abstandsmesser EN eine Entfernungsmessung, um einen Abstand während des Auftragens des Funktionalisierungswirkstoffes zu der Abgabevorrichtung in Form des Druckkopfes DK zu justieren oder einen Referenzpunkt zu bestimmen. Die Bestimmung des Referenzpunktes ermöglicht darüber hinaus auch eine Positionierung der dentalen Restauration in Form des Formkörpers FK oder des Trägers ST zu einem Nullpunkt.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar, insofern sie in den nachstehenden Ansprüchen definiert sind.

## Patentansprüche

1. Verfahren zur Funktionalisierung dentaler Restaurationen, bei dem folgende Schritte ausgeführt werden:
- Einsetzen eines Formkörpers in eine Aufnahme einer Appliziervorrichtung,
- Bereitstellen eines digitalen Datensatzes, der wenigstens einen Teilbereich der Oberfläche des dentalen Formkörpers repräsentiert,
- Auswählen einer Funktionalisierungsfläche innerhalb des Teilbereichs der Oberfläche des dentalen Formkörpers,
- Infiltration eines Volumens unter der Funktionalisierungsfläche mit einem Funktionalisierungswirkstoff, wobei die Infiltration mit dem Funktionalisierungswirkstoff durch gezieltes Bewegen der Aufnahme und/oder einer Abgabevorrichtung erfolgt und die Infiltration eine Massezunahme des Formkörpers bei im wesentlichen unveränderten Volumen des Formkörpers bewirkt,
- Entnahme des dentalen Formkörpers aus der Appliziervorrichtung, und
- Aktivieren des Funktionalisierungswirkstoffs in einem thermischen, optischen oder chemischen Vorgang.

2. Verfahren nach Anspruch 1, bei dem der Formkörper als poröse Keramik, offene Keramik, Weißkeramik, Grünkeramik, Grünling oder monolithischer Keramik bereitgestellt oder aus daraus bestehendem Halbzeug verarbeitet wird , mittels eines Pressverfahrens, mittels eines formgebenden Gießverfahrens oder in einem formfreien 3D-Druckverfahren hergestellt wird.

3. Verfahren nach einem der Ansprüche 1 bis 2, bei dem als Funktionalisierungswirkstoff ein Farbauftrag, ein lichthärtender Lack, ein Kaltsilan, eine Harzinfiltration oder ein oberflächlicher Auftrag von keramischen oder polymeren Lasuren, Metalloxiden oder seltene Erden zur Veränderung der optischen Eigenschaften applizierbar ist.

4. Verfahren nach einem der Ansprüche 1 bis 3, bei dem die Appliziervorrichtung als Abgabevorrichtung einen Druckkopf, einen Sprühkopf oder eine auf kontaktbehaftetem Drucken basierende Applikationseinheit aufweist.

5. Verfahren nach einem der Ansprüche 1 bis 4, bei dem der digitale Datensatz mittels einer Raumüberführung und lagerichtiger Zuordnung in die Appliziervorrichtung übernommen wird.

6. Verfahren nach einem der Ansprüche 1 bis 5, bei dem der digitale Datensatz über eine interne oder externe Messvorrichtung bestimmt wird, die taktil, optisch oder akustisch die Oberfläche des dentalen Formkörpers erfasst.

7. Verfahren nach einem der Ansprüche 1 bis 6, bei dem die Messvorrichtung als Kamera oder als dreidimensionaler Scanner ausgebildet ist.

8. Verfahren nach einem der Ansprüche 1 bis 7, bei dem zusätzlich auf der Oberfläche des Formkörpers eine Farbmessung mittels eines Digitalsystems durchgeführt wird.

9. Verfahren nach einem der Ansprüche 1 bis 8, bei dem eine Entfernungsmessung erfolgt, um einen Abstand während des Auftragens des Funktionalisierungswirkstoffes zu der Abgabevorrichtung zu justieren oder einen Referenzpunkt zu bestimmen.

10. Verfahren nach einem der Ansprüche 1 bis 9, bei dem ausgehend von dem digitalen Datensatz die Appliziervorrichtung definierte Flächen ansteuern und das Infiltrieren des Funktionalisierungswirkstoffes bezüglich der verwendeten Materialien, deren Stärken oder Dichten steuern kann.

11. Appliziervorrichtung zur Durchführung eines Verfahrens zur Funktionalisierung dentaler Restaurationen, insbesondere nach einem der Ansprüche 1 bis 10, umfassend eine Auflage zur Aufnahme eines teilbearbeiteten dentalen Formkörpers für die Herstellung einer dentalen Restauration und eine Abgabevorrichtung zum wenigstens abschnittsweisen räumlich vordefinierten Infiltrieren eines flüssigen Funktionalisierungswirkstoffs eines Volumens unter einer über einen digitalen Datensatz zugeordneten Oberfläche des dentalen Formkörpers, wobei die Infiltration eine Massezunahme des Formkörpers bei im wesentlichen unveränderten Volumen des Formkörpers bewirkt.

12. Appliziervorrichtung nach Anspruch 11, bei der die Auflage entlang mehrerer Achsen bewegbar, vorzugsweise entlang einer ersten Achse und einer zweiten Achse verschiebbar und entlang der zweiten Achse und/oder einer dritten Achse drehbar, ist und das Auftragen des Funktionalisierungswirkstoffs durch gezieltes Bewegen der Auflage erfolgt.

13. Appliziervorrichtung nach einem der Ansprüche 11 bis 12, bei der die Abgabevorrichtung entlang wenigstens einer weiteren Achse bewegbar ist und das Auftragen des Funktionalisierungswirkstoffs durch gezieltes Bewegen der Abgabevorrichtung erfolgt.

14. Softwareprogrammprodukt, das für einen Prozessor lesbare Befehle enthält, die geeignet sind, ein Verfahren gemäß einem der Ansprüche 1 bis 10 auszuführen.

15. Steuereinrichtung einer Appliziervorrichtung, die aus einem Speicher Befehle erhält, die geeignet sind, ein Verfahren gemäß einem der Ansprüche 1 bis 10 auszuführen.

## Claims

1. Method for functionalizing dental restorations, wherein the following steps are carried out:
- inserting a shaped body into a receiving portion of an application apparatus;
- providing a digital data set that represents at least a partial region of the surface of the dental shaped body;
- selecting a functionalization surface within the partial region of the surface of the dental shaped body;
- infiltrating a volume below the functionalization surface with an active functionalization substance, the infiltration with the active functionalization substance being carried out by moving the receiving portion and/or a dispensing apparatus in a targeted manner and the infiltration causing an increase in the mass of the shaped body while the volume of the shaped body remains substantially unchanged;
- removing the dental shaped body from the application apparatus; and
- activating the active functionalization substance in a thermal, optical or chemical process.

2. Method according to claim 1, wherein the shaped body is provided as a porous ceramic, open ceramic, white ceramic, green ceramic, green compact or monolithic ceramic or is processed from semi-finished products consisting thereof by means of a pressing method, by means of a shaping casting method or in a mold-free 3D printing method.

3. Method according to either of claims 1 or 2, wherein a color application, a light-curing varnish, a cold silane, a resin infiltration or a surface application of ceramic or polymeric glazes, metal oxides or rare earths can be applied as the active functionalization substance in order to change the optical properties.

4. Method according to any of claims 1 to 3, wherein the application apparatus has a print head, a spray head or an application unit based on contact printing as the dispensing apparatus.

5. Method according to any of claims 1 to 4, wherein the digital data set is transferred to the application apparatus by means of spatial transfer and assignment to the correct position.

6. Method according to any of claims 1 to 5, wherein the digital data set is determined via an internal or external measuring apparatus which detects the surface of the dental shaped body in a tactile, optical or acoustic manner.

7. Method according to any of claims 1 to 6, wherein the measuring apparatus is designed as a camera or as a three-dimensional scanner.

8. Method according to any of claims 1 to 7, wherein a color measurement is additionally carried out on the surface of the shaped body by means of a digital system.

9. Method according to any of claims 1 to 8, wherein a distance measurement is carried out in order to adjust, during the application of the active functionalization substance, a distance from the dispensing apparatus or to determine a reference point.

10. Method according to any of claims 1 to 9, wherein, starting from the digital data set, the application apparatus can select defined surfaces and can control the infiltration of the active functionalization substance with regard to the materials used, their strengths or densities.

11. Application apparatus for carrying out a method for functionalizing dental restorations, in particular according to any of claims 1 to 10, comprising a support for holding a partially processed dental shaped body for the production of a dental restoration and a dispensing apparatus for at least partially spatially predefined infiltration of a liquid active functionalization substance of a volume below a surface of the dental shaped body, which surface is assigned via a digital data set, wherein the infiltration causes an increase in the mass of the shaped article while the volume of the shaped article remains substantially unchanged.

12. Application apparatus according to claim 11, wherein the support is movable along a plurality of axes, preferably slidable along a first axis and a second axis and rotatable along the second axis and/or a third axis, and the active functionalization substance is applied by moving the support in a targeted manner.

13. Application apparatus according to either of claims 11 or 12, wherein the dispensing apparatus can be moved along at least one further axis and the active functionalization substance is applied by moving the dispensing apparatus in a targeted manner.

14. Software program product containing instructions that can be read by a processor and are suitable for carrying out a method according to any of claims 1 to 10.

15. Control device of an application apparatus which receives instructions from a memory which are suitable for carrying out a method according to any of claims 1 to 10.

## Revendications

1. Procédé de fonctionnalisation de prothèses dentaires, dans lequel les étapes suivantes sont mises en œuvre :
- insertion d'un corps moulé dans un logement d'un dispositif d'application,
- mise à disposition d'un ensemble de données numériques qui représente au moins une zone partielle de la surface du corps moulé dentaire,
- sélection d'une surface de fonctionnalisation à l'intérieur de la zone partielle de la surface du corps moulé dentaire,
- infiltration d'un volume sous la surface de fonctionnalisation avec un principe actif de fonctionnalisation, l'infiltration avec le principe actif de fonctionnalisation étant effectuée par déplacement ciblé du logement et/ou d'un dispositif distributeur, et l'infiltration provoquant une augmentation de la masse du corps moulé tandis que le volume du corps moulé reste sensiblement inchangé,
- retrait du corps moulé dentaire du dispositif d'application, et
- activation du principe actif de fonctionnalisation dans un processus thermique, optique ou chimique.

2. Procédé selon la revendication 1, dans lequel le corps moulé est mis à disposition sous forme de céramique poreuse, de céramique ouverte, de céramique blanche, de céramique crue, d'ébauche crue ou de céramique monolithique ou est transformé à partir d'un produit semi-fini constitué de celles-ci, et est fabriqué au moyen d'un procédé de pressage, d'un procédé de coulée par moulage ou dans un procédé d'impression 3D sans moule.

3. Procédé selon l'une des revendications 1 à 2, dans lequel un dépôt de couleur, un vernis photopolymérisable, un silane froid, une infiltration de résine ou un dépôt en surface de glaçures céramiques ou polymères, d'oxydes métalliques ou de terres rares peut être appliqué(e) en tant que principe actif de fonctionnalisation pour modifier les propriétés optiques.

4. Procédé selon l'une des revendications 1 à 3, dans lequel le dispositif d'application présente, en tant que dispositif distributeur, une tête d'impression, une tête de pulvérisation ou une unité d'application basée sur l'impression par contact.

5. Procédé selon l'une des revendications 1 à 4, dans lequel l'ensemble de données numériques est pris en charge dans le dispositif d'application au moyen d'un transfert spatial et d'une affectation en position correcte.

6. Procédé selon l'une des revendications 1 à 5, dans lequel l'ensemble de données numériques est déterminé par l'intermédiaire d'un dispositif de mesure interne ou externe qui détecte la surface du corps moulé dentaire de manière tactile, optique ou acoustique.

7. Procédé selon l'une des revendications 1 à 6, dans lequel le dispositif de mesure est conçu comme une caméra ou comme un scanner tridimensionnel.

8. Procédé selon l'une des revendications 1 à 7, dans lequel une mesure de couleur est en outre réalisée sur la surface du corps moulé au moyen d'un système numérique.

9. Procédé selon l'une des revendications 1 à 8, dans lequel une mesure de distance est effectuée afin d'ajuster un écart pendant le dépôt du principe actif de fonctionnalisation par rapport au dispositif distributeur ou de déterminer un point de référence.

10. Procédé selon l'une des revendications 1 à 9, dans lequel, à partir de l'ensemble de données numériques, le dispositif d'application peut activer des surfaces définies et peut commander l'infiltration du principe actif de fonctionnalisation en fonction des matériaux utilisés, de leurs résistances ou de leurs densités.

11. Dispositif d'application destiné à mettre en oeuvre un procédé de fonctionnalisation de prothèses dentaires, en particulier selon l'une des revendications 1 à 10, comprenant un support destiné à loger un corps moulé dentaire partiellement usiné pour la fabrication d'une prothèse dentaire, et un dispositif distributeur destiné à infiltrer de façon spatialement prédéfinie, au moins par sections, un principe actif de fonctionnalisation liquide d'un volume sous une surface du corps moulé dentaire affectée par l'intermédiaire d'un ensemble de données numériques, l'infiltration provoquant une augmentation de la masse du corps moulé tandis que le volume du corps moulé reste sensiblement inchangé.

12. Dispositif d'application selon la revendication 11, dans lequel le support peut être déplacé le long de plusieurs axes, de préférence peut coulisser le long d'un premier axe et d'un deuxième axe et est rotatif le long du deuxième axe et/ou d'un troisième axe, et le dépôt du principe actif de fonctionnalisation est effectué par déplacement ciblé du support.

13. Dispositif d'application selon l'une des revendications 11 à 12, dans lequel le dispositif distributeur peut être déplacé le long d'au moins un autre axe et le dépôt du principe actif de fonctionnalisation est effectué par déplacement ciblé du dispositif distributeur.

14. Produit de programme de logiciel contenant des instructions lisibles par un processeur, lesquelles instructions sont aptes à mettre en œuvre un procédé selon l'une des revendications 1 à 10.

15. Moyen de commande d'un dispositif d'application recevant des instructions à partir d'une mémoire, lesquelles instructions sont aptes à mettre en œuvre un procédé selon l'une des revendications 1 à 10.
